# EUROPEAN PATENT APPLICATION

(11) **EP 4 421 488 A1**
(43) Date of publication of application: **28.08.2024**
(21) Application number: 23158415.2
(22) Date of filing: 24.02.2023
(51) Int. Cl.: G01N 33/207, G01N 25/72, H01M 10/42, B23K 31/12

(54) **METHOD AND ARRANGEMENT FOR DEFINING QUALITY OF BUSBAR WELDINGS**

(71) Applicant: Valmet Automotive Oy, 23501 Uusikaupunki (FI)
(72) Inventor: Kulmala, Matti, 23501 Uusikaupunki (FI); Pesonen, Jarmo, 23501 Uusikaupunki (FI)
(74) Representative: Papula Oy

(57) **Abstract**

A method and an arrangement for defining the quality of busbar weldings, the method comprising
- providing (500) a holder (1) that comprises a set of temperature sensors (2). The temperature sensors (2) being arranged to a predetermined pattern (3) according to the welding spots (4) in the busbar (5). Placing (501) the holder (1) on the busbar (5) welded to terminals (6) of battery cells and pressing (502) the temperature sensors (2) by a contacting force against the busbar (5) so that they make a contact with the busbar (5) in measuring spots (7), heating up (503) the busbar (5), measuring (504) temperatures of the measuring spots (7) with the temperature sensors (2), creating (505) a measuring spot specific temperature response based on temperatures measured in the corresponding measuring spot, and defining (506) the welding quality of the welding spot (4) based on the measuring spot specific temperature response of the nearest measuring spot (7).

## Description

### BACKGROUND

The invention relates to a method for defining the quality of busbar weldings.

The invention further relates to an arrangement for defining the quality of busbar weldings.

The quality of busbar weldings plays an important role in operation of a battery. A battery may comprise dozens or hundreds of busbar weldings, and one poor welding among these may cause battery failure. The electrical conductivity of the poor welding is bad, which rises resistance and voltage drop thereof, causing rise of temperature. Thus, the quality of busbar weldings should be defined in the manufacturing of batteries, preferably as early stage of the manufacturing as possible. A problem with the defining the quality of busbar weldings is that it is difficult and burdensome to carry out. Furthermore, known methods based on thermal cameras may be unreliable due to often reflective surfaces of the busbars.

### BRIEF DESCRIPTION

Viewed from a first aspect, there can be provided a method for defining the quality of busbar weldings, the method comprising
- providing a holder,
- the holder comprising a set of temperature sensors,
- the temperature sensors being arranged to a predetermined pattern according to the welding spots in the busbar,
- placing the holder on the busbar welded to terminals of battery cells,
- pressing the temperature sensors by a contacting force against the busbar so that they make a contact with the busbar in measuring spots,
- heating up the busbar,
- measuring temperatures of the measuring spots with the temperature sensors,
- creating a measuring spot specific temperature response based on temperatures measured in the corresponding measuring spot, and
- defining the welding quality of the welding spot based on the measuring spot specific temperature response of the nearest measuring spot.

Thereby a method for defining the quality of busbar weldings that is quick and reliable and able to define also the quality of the weldings in glossy or reflective busbars may be achieved.

Viewed from a further aspect, there can be provided an arrangement for defining the quality of busbar weldings, the arrangement comprising a holder, comprising a set of temperature sensors arranged to a predetermined pattern according to the welding spots of the busbar, and each of the temperature sensors provided with a limiter device arranged for limiting the contacting force of the temperature sensor against the busbar, a data managing system for monitoring temperatures data measured by the temperature sensors, creating a measuring spot specific temperature response based on temperatures measured in the corresponding measuring spot, and defining the welding quality of the welding spot based on the measuring spot specific temperature response of the nearest measuring spot.

Thereby an arrangement for defining the quality of busbar weldings that is quick to use and provides reliable measurements also when defining the quality of the weldings in glossy or reflective busbars may be achieved.

The arrangement and the method are characterised by what is stated in the independent claims. Some other embodiments are characterised by what is stated in the other claims. Inventive embodiments are also disclosed in the specification and drawings of this patent application. The inventive content of the patent application may also be defined in other ways than defined in the following claims. The inventive content may also be formed of several separate inventions, especially if the invention is examined in the light of expressed or implicit sub-tasks or in view of obtained benefits or benefit groups. Some of the definitions contained in the following claims may then be unnecessary in view of the separate inventive ideas. Features of the different embodiments of the invention may, within the scope of the basic inventive idea, be applied to other embodiments.

Various embodiments of the first aspect may comprise at least one feature from the following paragraphs:
In one embodiment, the measured temperature response comprises the temperature reached in the measuring spot.

An advantage is that the temperature response is very easy to determine.

In one embodiment, the measured temperature response comprises rate of temperature change, that is temperature rising speed or temperature decreasing speed, or both, in the measuring spot.

An advantage is that the accuracy of the temperature response may be very high, since it is based on multiple measuring points or times of measuring. Additionally, it is possible to calculate the amount of heat energy created in the measuring spot, which way the margin of error in the temperature response may be reduced. This calculation may be done e.g. by calculating the area between the measured temperature curve and the time-axis (x-axis).

In one embodiment, the temperature is measured in a measuring spot between two successive welding spots, and the welding quality of said two successive welding spots are defined based on said measuring.

An advantage is that the number of the temperature sensors needed for defining the quality of the busbar may be reduced.

In one embodiment, the contacting force is set low enough for essentially avoiding movement or bending of the busbar towards the terminal.

An advantage is that measuring errors caused by the measurement arrangement may be avoided or at least essentially minimized.

In one embodiment, the temperature sensor is selected in group of a thermocouple (TC), a resistance temperature detector (RTD), a negative temperature coefficient (NTC) termistor, a temperature sensor IC. The temperature sensor is preferably RTD type of sensor, since it is accurate and has a simple structure.

In one embodiment, the busbar is heated up by high current for a predetermined heating time.

An advantage is that heating is quick and easily controlled.

In one embodiment, it is measured also the direct current internal resistance value of the busbar.

An advantage is that that the direct current internal resistance value may be used for confirming the measuring spot specific temperature responses.

In one embodiment, it is set a lower limit for the temperature for observing malfunction.

An advantage is that malfunction of the arrangement may be noticed.

In one embodiment, the limiter device comprises at least one of the following: a spring, a foam element, a pneumatic device, a hydraulic device. The limiter device comprises preferably a spring, such as a coil spring, since its stiffness can be selected very precisely, it is simple to manufacture for providing a desired travelling length, and it is easy to renew.

### BRIEF DESCRIPTION OF FIGURES

Some embodiments illustrating the present disclosure are described in more detail in the attached drawings, in which
Figure 1 is a schematic top view of a busbar arrangement,
Figure 2 is a schematic top view of an arrangement for defining the quality of busbar weldings of the busbar arrangement shown in Figure 1,
Figure 3 is another schematic view of the arrangement shown in Figure 2,
Figure 4 illustrates measuring spot specific temperature responses based on temperatures measured in two measuring spots,
Figure 5 illustrates a method for defining the quality of busbar weldings, and
Figure 6 illustrates another measuring spot specific temperature responses based on temperatures measured in two measuring spots.

In the figures, some embodiments are shown simplified for the sake of clarity. Similar parts are marked with the same reference numbers in the figures.

### DETAILED DESCRIPTION

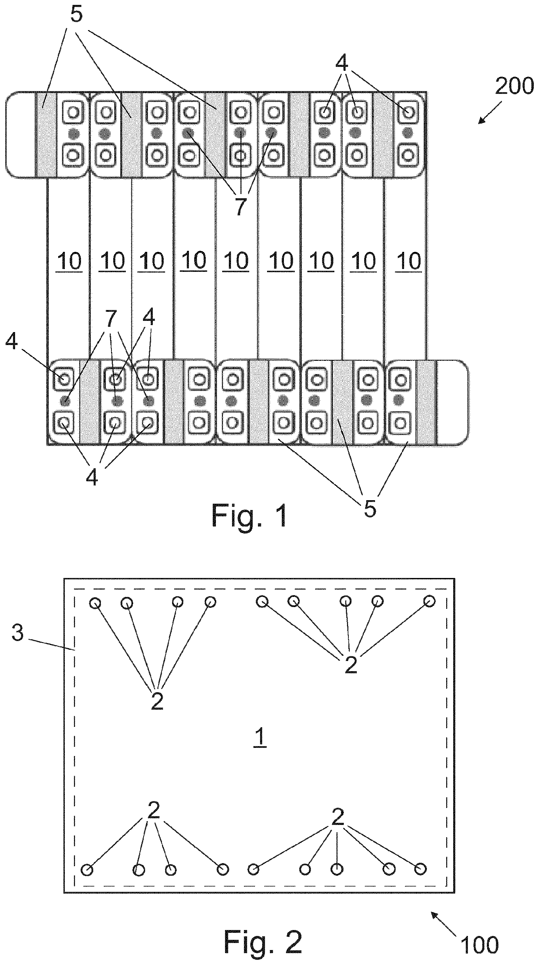

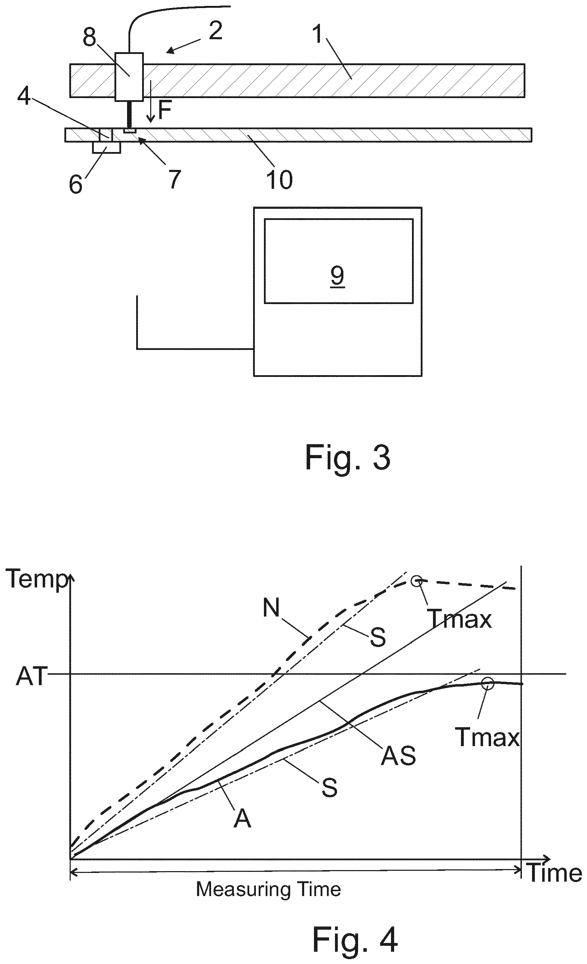

**Figure 1** is a schematic top view of a busbar arrangement, **Figure 2** is a schematic top view of an arrangement for defining the quality of busbar weldings of the busbar arrangement shown in Figure 1, and **Figure 3** is another schematic view of the arrangement shown in Figure 2.

In one embodiment, the busbar arrangement 200 comprises just one busbar 5. In one embodiment, the busbar arrangement 200 comprises plurality of busbars 5.

The busbar 5 is of electrically conductive metal, such as of aluminium or copper. The method and the arrangement described herein are insensitive to surface characteristics of the busbar. Thus, the surface of the busbar 5 may have any finish, e.g. the surface may be shiny or glossy metallic surface, or matt metallic surface, or painted glossy or matt surface, for instance.

The busbar 5 is arranged to connect electrically two or more cell terminals (shown in Figure 3) of battery cells 10 in a same electrical polarity. The connection between the busbar 5 and the cell terminal is made by welding spots 4.

In one embodiment, the welding spot 4 is laser welded.

In one embodiment, the welding spot 4 is ultrasonic welded.

In one embodiment, the welding spot 4 is MIG (Metal Inert Gas) welded.

In one embodiment, the welding spot 4 is TIG (Tungsten Inert Gas) welded.

In one embodiment, the welding spot 4 is a point-like spot.

In one embodiment, the welding spot 4 is a welding line. The welding line may be a continuous line, or also a series of point-like spots in close succession.

For example, the busbar arrangement 200 shown in Figure 1 comprises ten (10) busbars 5. Most of said busbars are connecting two cell terminals 10, but the busbar arranged in Figure 1 at the upper left and at the lower right ends are connecting the busbar arrangement 200 to electrical circuit of the battery.

The battery cells 10 and the busbar arrangement 200 are parts of a battery (not shown). In one embodiment, the battery is a drive battery of a fully or partly electricity-driven vehicle, such as a car. In one embodiment, the battery is a battery of a stationary electricity source.

The arrangement 100 comprises a holder 1 that is made of e.g. a metal or plastic plate, a pipe or profile frame, etc. A set of temperature sensors 2 is arranged and attached to the holder 1 in a predetermined pattern 3. The temperature sensors 2 can be e.g. attached to openings or boreholes that extent through the holder 1.

The predetermined pattern 3 is made according to locations of the welding spots 4 of the busbar 5 to be tested, so that when the holder 1 is arranged on the busbar arrangement 200, the temperature sensors 2 - more precisely their measuring head or probe protruding from the holder - touch with measuring spots 7 on the busbar(s). The measuring spots 7 are spots or points of the busbar 5 the temperature of which are to be measured with temperature sensors 2.

The holder 1 shown in Figure 1 has a one-part structure that holds all the temperature sensors 2 of the arrangement 100. In another embodiment, the arrangement 100 comprises two, three, four or even more holders into which the temperature sensors 2 are distributed in a predetermined pattern. A reason for using two or more holders 1 may be e.g. the structure of the busbar arrangement 200.

In order to carry out temperature measurements, the holder 1 is arranged on the busbar 5 so that the temperature sensors 2 are positioned on the measuring spots 7. This can be performed by e.g. an actuator the movements of which are controlled by an automatized controlling system.

Figure 1 is showing a version of the busbar arrangement 200 wherein the welding spots 4 are in pairs. The nearest measuring spot 7 lies between said pair of welding spots 4. Thus, the number of the measuring spots 7 is half of the number of the welding spots 4. In the method it is measured the temperature in the measuring spot 7 between two successive welding spots 4, i.e between said pair of welding spots, and the welding quality of said two successive welding spots 4 is defined based on said measuring.

In one embodiment, the nearest measuring spot is on or above the welding spot 4.

It is to be noted, however, as that size, shape, and number of welding spots vary, also the holder size, shape, and placement of the temperature sensors vary. For instance, it is not necessary that the side profile of the holder 1 is not necessary plane-like or two dimensional as shown in Figure 3; instead, the side profile may be three dimensional. There may be a measuring spot 7 dedicated only to one welding spot 4 for measuring the measuring spot specific temperature response for said welding spot, for instance. In one embodiment, all or at least most of the measuring spots 7 are dedicated to measure the measuring spot specific temperature response for only one welding spot 4.

In one embodiment, the temperature sensor 2 comprises a thermocouple (TC).

In one embodiment, the temperature sensor 2 comprises a resistance temperature detector (RTD).

In one embodiment, the temperature sensor 2 comprises a negative temperature coefficient (NTC) thermistor.

In one embodiment, the temperature sensor 2 comprises a temperature sensor IC (integrated circuit).

In one embodiment, each of the temperature sensors 2 is provided with a limiter device 8 (shown in Figure 3). The limiter device 8 is arranged for limiting the contacting force F of the temperature sensor 2 against the busbar 5. The contacting force F shall be low enough that movement or bending of the busbar 5 towards the terminal 6 is essentially avoided. Thus, the allowable contacting force F is specific for the busbar arrangement and the characteristics of the terminals, for instance.

In one embodiment, the limiter device 8 comprises a spring, such as a coil spring or torsion spring.

In one embodiment, the limiter device 8 comprises a foam element, such as polyurethane spring.

In one embodiment, the limiter device 8 comprises a pneumatic device, such as an air spring.

In one embodiment, the limiter device 8 comprises a hydraulic device, such as a hydraulic spring.

For carrying out the method and measuring the measuring spot specific temperature responses, the busbar 5 is heated up. In one embodiment, the busbar 5 is heated up by high current that is fed thereto a predetermined heating time. In one embodiment, the predetermined heating time is selected in range of 5 seconds to 30 seconds, preferably in range of 8 seconds to 15 seconds. In one embodiment, the predetermined heating time is about 10 seconds.

In one embodiment, the temperature measurement is carried out during the heating. In one embodiment, the temperature measurement is started before starting the heating for recording ambient temperature or temperature of the measuring spot prior to the heating.

In one embodiment of the method, the heating is combined with a DCIR (direct current internal resistance) test that is designed to determine the internal resistance of the battery. In other words, during the DCIR test a high current is charged or discharged to or from the battery via the busbar(s). During this test the measuring spot specific temperature responses are measured. An advantage of this embodiment is that the method does not extend or lengthen the manufacturing and/or testing time of the battery, but the method can be run simultaneously with another manufacturing/testing step.

A data managing system 9 is arranged for monitoring temperatures data measured by the temperature sensors 2. The data managing system 9 may comprise a processor (CPU) with a memory configured to store program code and dynamic data. In one embodiment, the data managing system 9 comprises a personal computer or laptop. Digital inputs corresponding to the measurement results are applied to the processor.

The data managing system 9 creates a measuring spot specific temperature response based on temperatures measured in the corresponding measuring spot, and defines the welding quality of the welding spot 4 based on the measuring spot specific temperature response of the nearest measuring spot 7.

In one embodiment, if any of the welding spots 4 has welding quality not fulfilling predetermined quality limits, the data managing system 9 may output a corresponding "welding quality not acceptable" or similar indication through a visual, an audible or similar signal. The indication preferably indicates the location of the not acceptable welding to an operator of the data managing system 9. An indication "welding quality of all the weldings are acceptable" may also be generated if all the weldings fulfil the predetermined quality limits.

In one embodiment, the arrangement and the method comprise further sensors or measurements, such as measuring the direct current internal resistance value of the busbar 5.

In one embodiment, a lower limit for the temperature measured is set for observing malfunction of the arrangement. If the temperature is lower than said limit, the data managing system 9 outputs a corresponding alarm.

**Figure 4** illustrates measuring spot specific temperature responses based on temperatures measured in two measuring spots.

The curve "A" represents an acceptable measuring spot where the temperature response is acceptable, and thus the welding(s) in the welding spot(s) which the acceptable measuring spot is representing is/are acceptable.

The curve "N" represents a non-acceptable measuring spot where the temperature response is not acceptable, and thus the welding(s) in the welding spot(s) which the non-acceptable measuring spot is representing is/are not acceptable.

In one embodiment, the measured temperature response comprises the maximum temperature Tmax reached in the measuring spot 7. In this embodiment, it is predetermined an acceptable maximum temperature AT. If the temperature Tmax reached during the measuring time rises over said acceptable maximum temperature AT, the quality of the corresponding welding is not acceptable. On the other hand, if the temperature during the measuring time stays under said acceptable maximum temperature AT, or if it is not more than said acceptable maximum temperature AT, the quality of the corresponding welding is acceptable.

In one embodiment, the measuring time is selected in range of 8 - 25 seconds, such as 14 seconds.

In one embodiment, the measured temperature response comprises rate of temperature change as the temperature rising speed (curve) in the measuring spot 7. In this embodiment, it is predetermined an acceptable slope or angular coefficient AS for the temperature rising speed during the measurement. If the temperature rising speed or its slope S during the measuring time is faster than said acceptable slope or angular coefficient AS, the quality of the corresponding welding is not acceptable. On the other hand, if the temperature rising speed during the measuring is not more than said slope or angular coefficient AS, the quality of the corresponding welding is acceptable.

Typically, the temperature of the busbar continues to raise some seconds after the heating thereof is stopped. Thus in one embodiment, the measuring time is continued momentarily after the heating is stopped.

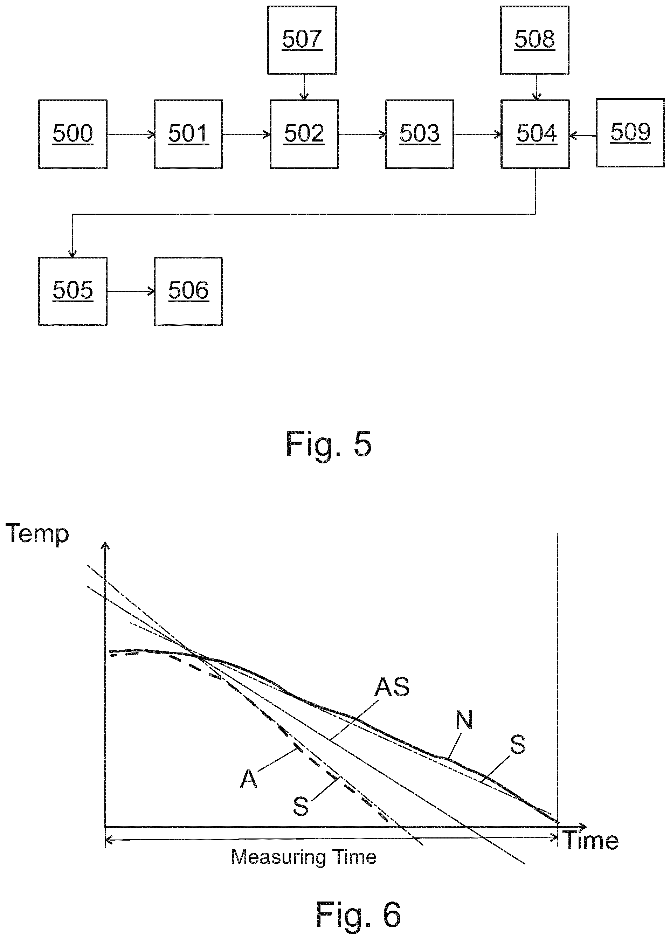

**Figure 6** illustrates another measuring spot specific temperature responses based on temperatures measured in two measuring spots. In this embodiment, the measured temperature response is measured after the heating of the busbar is stopped, i.e. the measured temperature response comprises temperature decreasing speed. The measuring time may be e.g. 10 seconds. Also here it is defined an acceptable slope or angular coefficient AS, but now for the temperature decreasing speed during the measurement. If the temperature decreasing speed or its slope S during the measuring time is faster than said acceptable slope or angular coefficient AS, the quality of the corresponding welding is not acceptable. On the other hand, if the temperature decreasing speed during the measuring is not more than said slope or angular coefficient AS, the quality of the corresponding welding is acceptable.

In one embodiment, the measured temperature response is measured during the heating of the busbar and continued after the heating is stopped. Thus, it is measured raising temperatures (such as shown in Figure 4) and additionally decreasing temperatures (such as shown in Figure 6). This embodiment may give further information about the quality of the busbar weldings.

In one embodiment, the rate of temperature change and the corresponding slope or angular coefficient is determined over the whole measuring time. In another embodiment, the rate of temperature change and the corresponding slope or angular coefficient is determined in certain phase or part of the measuring time. For instance, in one embodiment the angular coefficient is based on temperature measurements measured during first 1/3 of the measuring time.

**Figure 5** illustrates a method for defining the quality of busbar weldings. First it is provided 500 a holder that comprises a set of temperature sensors that are arranged to a predetermined pattern according to the welding spots in the busbar.

The holder is placed 501 on the busbar welded to terminals of battery cells.

The temperature sensors are pressed 502 by a contacting force against the busbar so that they make a contact with the busbar in measuring spots close the welding spots.

The busbar is heated up 503 and temperatures of the measuring spots are measured 504 with the temperature sensors in the holder.

A measuring spot specific temperature response is created 505 for each of the measuring spots based on temperatures measured in the corresponding measuring spot.

The welding quality of the welding spot is defined 506 based on the measuring spot specific temperature response of the nearest measuring spot.

In one embodiment, the contacting force is set 507 so low that the contacting force does not essentially move or bend the busbar towards the terminal.

In one embodiment, it is measured 508 also the direct current internal resistance value of the busbar 5.

In one embodiment, it is set 509 a lower limit or result for the temperature measurement for observing malfunction of the arrangement or the temperature sensor.

The invention is not limited solely to the embodiments described above, but instead many variations are possible within the scope of the inventive concept defined by the claims below. Within the scope of the inventive concept the attributes of different embodiments and applications can be used in conjunction with or replace the attributes of another embodiment or application.

The drawings and the related description are only intended to illustrate the idea of the invention. The invention may vary in detail within the scope of the inventive idea defined in the following claims.

### REFERENCE SYMBOLS

- 1: holder
- 2: temperature sensor
- 3: pattern
- 4: welding spot
- 5: busbar
- 6: terminal
- 7: measuring spot
- 8: limiter device
- 9: data managing system
- 10: battery cell

- 100: arrangement
- 200: busbar arrangement
- 500-: method steps

- A: acceptable welding spot
- AS: acceptable slope
- AT: acceptable maximum temperature
- F: pressing force
- N: non-acceptable measuring spot
- S: slope
- Tmax: maximum temperature

## Claims

1. A method for defining the quality of busbar weldings, the method comprising
- providing (500) a holder (1)
- the holder (1) comprising a set of temperature sensors (2),
- the temperature sensors (2) being arranged to a predetermined pattern (3) according to the welding spots (4) in the busbar (5),
- placing (501) the holder (1) on the busbar (5) welded to terminals (6) of battery cells,
- pressing (502) the temperature sensors (2) by a contacting force against the busbar (5) so that they make a contact with the busbar (5) in measuring spots (7),
- heating up (503) the busbar (5),
- measuring (504) temperatures of the measuring spots (7) with the temperature sensors (2),
- creating (505) a measuring spot specific temperature response based on temperatures measured in the corresponding measuring spot, and
- defining (506) the welding quality of the welding spot (4) based on the measuring spot specific temperature response of the nearest measuring spot (7).

2. The method as claimed in claim 1, wherein
- the measured temperature response comprises the temperature reached in the measuring spot (7).

3. The method as claimed in claim 1 or 2, wherein
- the measured temperature response comprises rate of temperature change in the measuring spot (7).

4. The method as claimed in any of the preceding claims, comprising
- measuring the temperature in a measuring spot (7) between two successive welding spots (4), and
- defining the welding quality of said two successive welding spots (4) based on said measuring.

5. The method as claimed in any of the preceding claims, wherein
- the welding spot (4) is laser welded or ultrasonic welded.

6. The method as claimed in any of the preceding claims, comprising
- setting (507) the contacting force (F) low enough for essentially avoiding movement or bending of the busbar (5) towards the terminal (6).

7. The method as claimed in any of the preceding claims, wherein
- the temperature sensor (2) is selected in group of a thermocouple (TC), a resistance temperature detector (RTD), a negative temperature coefficient (NTC) termistor, a temperature sensor IC.

8. The method as claimed in any of the preceding claims, comprising
- heating up the busbar (5) by high current for a predetermined heating time.

9. The method as claimed in any of the preceding claims, comprising
- measuring (508) also the direct current internal resistance value of the busbar (5).

10. The method as claimed in any of the preceding claims, comprising
- setting (509) a lower limit for the temperature for observing malfunction.

11. The method as claimed in any of the preceding claims, wherein
- the busbar (5) is of aluminium or copper.

12. An arrangement (100) for defining the quality of busbar weldings, the arrangement comprising
- a holder (1), comprising
- a set of temperature sensors (2) arranged to a predetermined pattern (3) according to the welding spots (4) of the busbar (5), and
- each of the temperature sensors (2) provided with a limiter device (8) arranged for limiting the contacting force (F) of the temperature sensor (2) against the busbar,
- a data managing system (9) for
- monitoring temperatures data measured by the temperature sensors,
- creating a measuring spot specific temperature response based on temperatures measured in the corresponding measuring spot, and
- defining the welding quality of the welding spot based on the measuring spot specific temperature response of the nearest measuring spot.

13. The arrangement as claimed in claim 12, wherein
- the temperature sensor (2) comprises a thermocouple (TC), a resistance temperature detector (RTD), a negative temperature coefficient (NTC) termistor, a temperature sensor IC.

14. The arrangement as claimed in claim 12 or 13, wherein
- the limiter device (8) comprises at least one of the following: a spring, a foam element, a pneumatic device, a hydraulic device.

15. The arrangement as claimed in any of claims 12-14, wherein
- the data managing system (9) is arranged to define the welding quality of the welding spot (4) based on at least one of the temperature reached in the measuring spot (7) and the rate of temperature change in the measuring spot (7) .
